# EUROPEAN PATENT SPECIFICATION

(11) **EP 3 877 514 B1**
(45) Date of publication and mention of the grant of the patent: **17.06.2026**
(21) Application number: 19883241.2
(22) Date of filing: 07.11.2019
(51) Int. Cl.: C12N 5/10, C12N 15/09, A61K 35/17, A61P 35/00

(54) **COMPOSITIONS AND METHODS FOR TREATING CANCER**
ZUSAMMENSETZUNGEN UND VERFAHREN ZUR BEHANDLUNG VON KREBS
COMPOSITIONS ET PROCÉDÉS POUR LE TRAITEMENT DU CANCER

(30) Priority: 08.11.2018 US 201862757383 P
(43) Date of publication of application: 15.09.2021
(73) Proprietor: IN8bio, Inc., New York, NY 10118 (US); The UAB Research Foundation, Birmingham, AL 35233 (US)
(72) Inventor: LAMB, Lawrence S., Jr., Birmingham, AL 35242 (US); HO, William, New York, NY 10001 (US)
(74) Representative: Kilburn & Strode LLP
(86) International application number: PCT/US2019/060231
(87) International publication number: WO 2020/097306

(56) References cited:
- WO-A1-2012/141990
- WO-A1-2016/087871
- WO-A1-2018/035413
- US-A1- 2018 250 337
- LAMB L S ET AL: "Engineered Drug Resistant γδ T Cells Kill Glioblastoma Cell Lines during a Chemotherapy Challenge: A Strategy for Combining Chemo- and Immunotherapy", PLOS ONE, vol. 8, no. 1, E51805, 11 January 2013 (2013-01-11), XP055127744, ISSN: 1932-6203, DOI: 10.1371/journal.pone.0051805
- ROCHLIN K ET AL: "Chemotherapy resistant gamma delta T-cell immunotherapy can leverage synergistic ligand expression through combinational chemotherapy and PARP-inhibitor use to enhance tumor cell recognition & killing", JOURNAL FOR IMMUNOTHERAPY OF CANCER, vol. 9, no. Suppl 2, 158, November 2021 (2021-11-01), 36th Annual Meeting of the Society for Immunotherapy of Cancer; virtual; 11-14 November 2021, pages A168 - A168, XP055964700, DOI: 10.1136/jitc-2021-SITC2021.158
- JONES A ET AL: "Enhancing glioblastoma cell stress response to improve gamma-delta T-cell immunotherapy", NEURO-ONCOLOGY, vol. 23, no. Supplement_6, EXTH-25, 12 November 2021 (2021-11-12), 26th Annual Meeting of the Society for Neuro-Oncology; Boston, MA, USA; 18-21 November 2021, pages vi168, XP055964705, ISSN: 1522-8517, DOI: 10.1093/neuonc/noab196.664

## Description

### RELATED APPLICATION

This application claims the benefit of U.S. Provisional Application No. 62/757,383, filed November 8, 2018.

### BACKGROUND OF THE INVENTION

Gamma-delta (γδ) T cells are an important subset of T lymphocytes as they can recognize a broad range of antigens without antigen priming and the presence of major histocompatibility complex (MHC) molecules. They can attack target cells directly through their cytotoxic activity or indirectly through the activation of other immune cells. γδ T-cell functional responses are induced upon several factors including the recognition of stress antigens, which promotes cytokine production and regulates pathogen clearance, inflammation, and tissue homeostasis in response to stress.

Both human γδ T-cell subsets exhibit a cytotoxic potential that is induced through, for example, the expression of cell surface receptors [i.e., γδ TCR (T-cell receptor) and NKG2D (natural killer group 2D) and is mediated in part by the release of soluble mediators (i.e., perforin and granzymes). γδ T-cells can produce granulysin, which is a potent anti-microbial protein, and express ligands such as CD95L and tumor necrosis factor-related apoptosis-inducing ligand, which engage several death receptors on target cells. In addition, γδ T-cells can kill their targets indirectly through antibody-dependent cellular cytotoxicity (ADCC) in a CD16-dependent mechanism. Other molecules such as DNAM-1 (DNAX accessory molecule-1), leukocyte function-associated antigen-1, and the co-stimulatory receptor CD27 are also involved in γδ T-cell activation and cytotoxicity.

Human γδ T cells can also exhibit an antigen-presenting capacity. Similar to dendritic cells (DCs), blood Vγ9Vδ2 T cells are able to respond to signals from microbes and tumors and prime CD4⁺ and CD8⁺ T cells. γδ T-APCs are believed to cross-present antigens directly to CD8⁺ T cells. The intracellular protein degradation and endosomal acidification are significantly delayed in γδ T cells in comparison to monocyte-derived DCs. The antigens are transported across IRAP (Insulin-Regulated Amino Peptidase)-positive early and late endosomes, and their processing consists of an export to the cytosol for degradation by the proteasome before being imported into a MHC-I-loading compartment. Activated γδ T cells are able to phagocytose tumor antigens and apoptotic or live cancer cells possibly through the scavenger receptor CD36 in a C/EBPα (CCAAT/enhancer-binding protein α)-dependent mechanism and mount a tumor antigen-specific CD8⁺ T-cell response. γδ T cells can also induce DC maturation through TNF-α production. Overall, γδ T cells can process a wide range of antigens for presentation and stimulate other immune cells. Therefore, γδ T-cells implication in response to infections or cancer may be leveraged to design new strategies in order to improve clinical response of human γδ T cell-based immunotherapy. For instance, WO 2018/035413A1 combines chemotherapy and immune checkpoint inhibitors with γδ T cells engineered to be resistant to said chemotherapy.

The Poly(ADP-ribose) polymerase-1 (PARP-1) has been implicated in multiple cellular processes such as DNA damage repair (DDR), apoptosis, and genome stability. Inhibitors of DDR, including, but not limited to, PARP inhibitors, result in genotoxic stress, local antigen release, and other immune mechanisms resulting in systemic antitumor response including recruitment of natural killer (NK) cells and CD8+ cells and γδ T cells. Genotoxic stress and stalled DNA replication forks resulting from DDR repair inhibition using an agent such as a PARP inhibitor are believed to induce expression of ligands for the NKG2D receptor. It is also believed that genomic instability resulting from DDR repair inhibition results in increased tumor mutational burden, neoantigens and/or activation of the stimulation of interferon genes (STING) pathway and overall increased tumor immunogenicity.

Increased tumor immunogenicity (e.g. increased upregulation of ligands for the NKG2D receptor) resulting from DDR inhibition is uniquely conducive to γδ T cell-mediated tumor immunosurveillance, and ultimately tumor cell killing by γδ T cells. The combined effects of DDR inhibition with human γδ T cell-based immunotherapy provides other numerous clinical advantages. For example, PARP inhibitors delivered by oral or intravenous administration to a patient are associated with symptoms such as nausea and fatigue, can be particularly troublesome and affect quality of life. And possible discontinuation of the PARP inhibitor may be indicated. Human γδ T cell-based immunotherapy combined with PARP inhibitors can avoid toxicities associated with PARP inhibitors by lowering doses necessary for PARP inhibitors while potentiating tumor killing.

### SUMMARY OF THE INVENTION

The invention provides a composition comprising a population of γδ T cells, for use in a method for treating cancer in a patient in need thereof, the method comprising,
i) administering to said patient a therapeutically effective amount of said composition;
ii) administering to said patient a therapeutically effective amount of a chemotherapeutic agent; and
iii) administering to said patient a therapeutically effective amount of a DDR inhibitor, wherein the DDR inhibitor is a PARP inhibitor.

Preferred features of the invention are set out in the dependent claims herein.

The disclosure provides combination therapies for treating cancer comprising compositions and methods for γδ T cell immunotherapy in combination DDR inhibitors, including but not limited to PARP inhibitors (PARPi). Preferably, the combination of γδ T cell immunotherapy and PARP inhibitors for the treatment of cancer further includes combinations with other immunotherapies such as immune checkpoint (ICP) blockade therapy and/or therapy with DNA damaging agents such as cytotoxic chemotherapeutic agents. Preferably, when the combination of γδ T cell immunotherapy and DDR inhibitor therapy further include one or more chemotherapeutic agents, the γδ T cells are genetically modified to impart resistance to the one or more chemotherapeutic agents used in the therapy.

Preferably, the disclosure provides methods for the treatment of cancer in a patient in need thereof comprising, i) administering to a patient a composition comprising a therapeutically effective amount of γδ T cells that are genetically engineered to be resistant to at least one chemotherapeutic agent; ii) administering to a patient a therapeutically effective amount of a chemotherapeutic agent; and iii) administering to a patient a therapeutically effective amount of a DDR inhibitor. Preferably the DDR inhibitor is a PARP inhibitor. Preferably, the PARP inhibitor is administered prior to administering the chemotherapeutic agent and prior to administering the composition comprising the genetically engineered γδ T cell. Preferably, the PARP inhibitor is administered about 1 day to about 21 days prior to administering the chemotherapeutic agent. Preferably, the genetically engineered γδ T cells is administered about 8 hours to about 72 hours after administration of the chemotherapeutic agent. Preferably, the genetically engineered γδ T cells are administered about 12 hours to about 36 hours after administration of the chemotherapeutic agent. Preferably, the genetically engineered γδ T cells is administered about 24 hours after administration of the chemotherapeutic agent. Preferably, the chemotherapeutic agent is an alkylating agent; a metabolic antagonist; a DNA demethylating agent; a substituted nucleotide; a substituted nucleoside; an antitumor antibiotic; a plant-derived antitumor agent or a nitrosourea. Preferably the chemotherapeutic agent is selected from cisplatin; carboplatin; etoposide; methotrexate (MTX); trimethotrexate (TMTX); temozolomide; dacarbazine (DTIC), raltitrexed; S-(4-Nitrobenzyl)-6-thioinosine (NBMPR); 6-benzyguanidine (6-BG); a nitrosourea (rabinopyranosyl-N-methyl-N-nitrosourea (Aranose), Carmustine (BCNU, BiCNU), Chlorozotocin, Ethylnitrosourea (ENU), Fotemustine, Lomustine (CCNU), Nimustine, N-Nitroso-N-methylurea (NMU), Ranimustine (MCNU), Semustine, Streptozocin (Streptozotocin)); cytarabine; camptothecin; and a therapeutic derivative of any thereof. Preferably, the γδ T-cells have been genetically modified to encode alkyl guanine transferase (AGT), P140KMGMT, O⁶ methylguanine DNA methyltransferase (MGMT), L22Y-DHFR, thymidylate synthase, dihydrofolate reductase, or multiple drug resistance-1 protein (MDR1). Preferably, the γδ T-cells have been genetically modified to be resistant to at least two chemotherapeutic agents selected from: is an alkylating agent; a metabolic antagonist; a DNA demethylating agent; a substituted nucleotide; a substituted nucleoside; an antitumor antibiotic; a plant-derived antitumor agent and a nitrosurea. Preferably, the γδ T-cells have been genetically modified to be resistant to at least two chemotherapeutic agents selected from cisplatin; carboplatin; etoposide; methotrexate (MTX); trimethotrexate (TMTX); temozolomide; dacarbazine (DTIC), raltitrexed; S-(4-Nitrobenzyl)-6-thioinosine (NBMPR); 6-benzyguanidine (6-BG); a nitrosourea (rabinopyranosyl-N-methyl-N-nitrosourea (Aranose), Carmustine (BCNU, BiCNU), Chlorozotocin, Ethylnitrosourea (ENU), Fotemustine, Lomustine (CCNU), Nimustine, N-Nitroso-N-methylurea (NMU), Ranimustine (MCNU), Semustine, Streptozocin (Streptozotocin)); cytarabine; camptothecin; and a therapeutic derivative of any thereof. Preferably, the chemotherapeutic agent is TMZ, methotrexate, DTIC, BCNU, CCNU, MCNU, NMU or ENU.

### BRIEF DESCRIPTION OF THE DRAWINGS

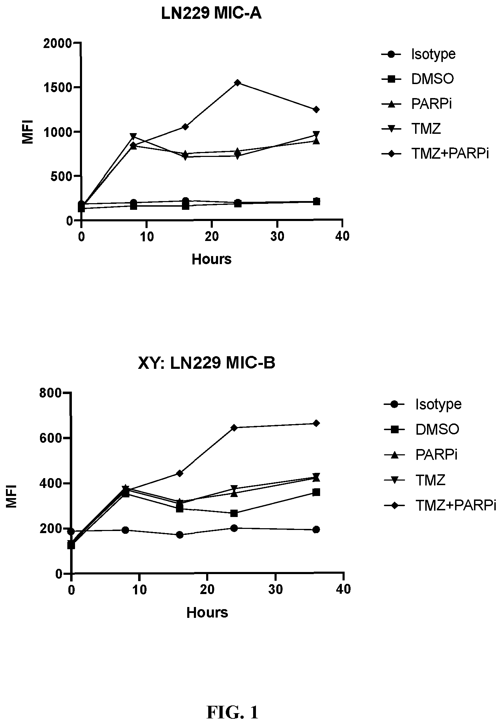

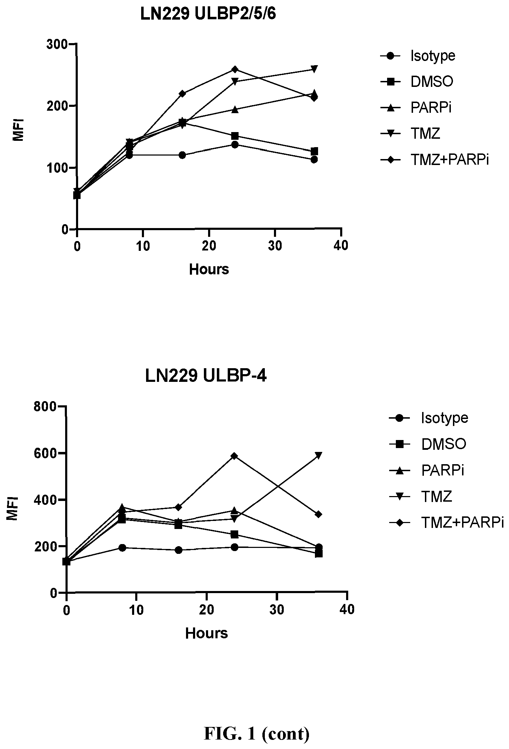

Figure 1 shows four, line graphs comparing the expression at selected time points of natural killer group 2D ligands (NKG2DL) on LN229 glioblastoma cell lines exposed to olaparib (5 or 10 mM) alone, olaparib (an exemplary PARP inhibitor)+ 200mg/mL TMZ, or media alone (RPMI + 10% FBS medium) for 4 hours and viability measured at initiation and 8, 16, 24, and 36 hours post treatment.

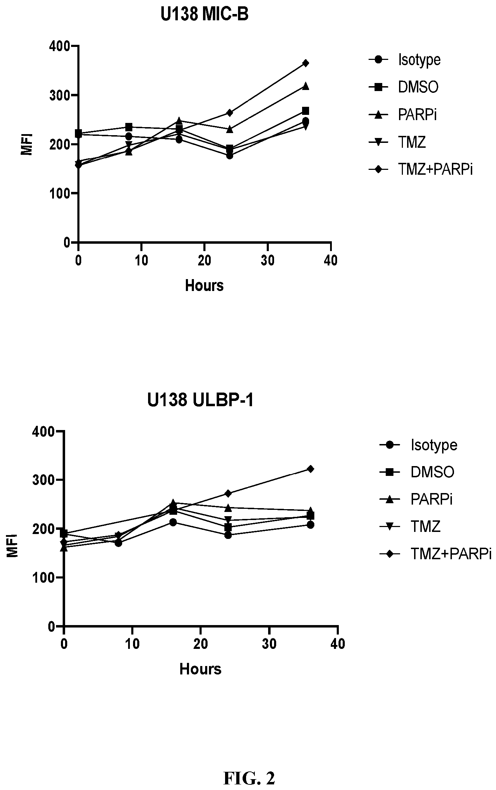

Figure 2 shows two, line graphs comparing the expression at selected time points of natural killer group 2D ligands (NKG2DL) on U138 glioblastoma cell lines exposed to exposed to olaparib (5 or 10 mM) alone, olaparib (an exemplary PARP inhibitor)+ 200mg/mL TMZ, or media alone (RPMI + 10% FBS medium) for 4 hours and viability measured at initiation and 8, 16, 24, and 36 hours post treatment.

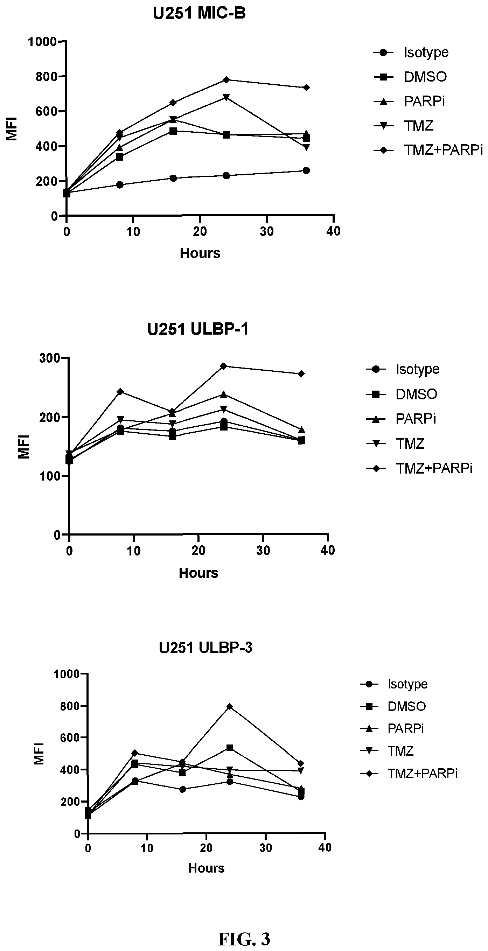

Figure 3 shows three, line graphs comparing the expression at selected time points of natural killer group 2D ligands (NKG2DL) on U251 glioblastoma cell lines exposed to exposed to olaparib (5 or 10 mM) alone, olaparib (an exemplary PARP inhibitor)+ 200mg/mL TMZ, or media alone (RPMI + 10% FBS medium) for 4 hours and viability measured at initiation and 8, 16, 24, and 36 hours post treatment.

### DETAILED DESCRIPTION OF THE INVENTION

### Definitions

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

As used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt. % to about 5 wt. %, but also include individual concentrations (e.g., 1%, 2%, 3%, and 4%) and the sub-ranges (e.g., 0.5%, 1.1%, 2.2%, 3.3%, and 4.4%) within the indicated range. The term "about" can include ±1%, ±2%, ±3%, ±4%, ±5%, ±6%, ±7%, ±8%, ±9%, or ±10%, or more of the numerical value(s) being modified. In addition, the phrase "about 'x' to 'y'" includes "about 'x' to about 'y'".

By "administration" is meant introducing a compound, biological materials including a cell population, or a combination thereof, of the present invention into a human or animal subject. One preferred route of administration of the compounds is intravenous. Other preferred routes of administration of the compounds may be intraperitoneal or intrapleural, or via a catheter to the brain. However, any route of administration, such as oral, topical, subcutaneous, peritoneal, intra-arterial, inhalation, vaginal, rectal, nasal, introduction into the cerebrospinal fluid, or instillation into body compartments can be used. Direct injection into a target tissue site such as a solid tumor is also contemplated.

The term "cancer", as used herein, shall be given its ordinary meaning, as a general term for diseases in which abnormal cells divide without control. Cancer cells can invade nearby tissues and can spread through the bloodstream and lymphatic system to other parts of the body. When normal cells lose their ability to behave as a specified, controlled and coordinated unit, a tumor is formed. Generally, a solid tumor is an abnormal mass of tissue that usually does not contain cysts or liquid areas (some brain tumors do have cysts and central necrotic areas filled with liquid). A single tumor may even have different populations of cells within it, with differing processes that have gone awry. Solid tumors may be benign (not cancerous), or malignant (cancerous). Different types of solid tumors are named for the type of cells that form them. Examples of solid tumors are sarcomas, carcinomas, and lymphomas. Leukemias (cancers of the blood) generally do not form solid tumors.

The term "reducing a tumor" as used herein refers to a reduction in the size or volume of a tumor mass, a decrease in the number of metastasized tumors in a subject, a decrease in the proliferative status (the degree to which the cancer cells are multiplying) of the cancer cells, and the like.

The term "chemotherapeutic agent" as used herein refers to a compound or a derivative thereof that can interact with a cancer cell, thereby reducing the proliferative status of the cell and/or killing the cell for example, by impairing cell division or DNA synthesis, or by damaging DNA, effectively targeting fast dividing cells. Examples of chemotherapeutic agents include, but are not limited to, alkylating agents (e.g., cyclophosphamide, ifosfamide); metabolic antagonists (e.g., methotrexate (MTX), 5-fluorouracil or derivatives thereof); a substituted nucleotide; a substituted nucleoside; DNA demethylating agents (also known as antimetabolites; e.g., azacitidine); antitumor antibiotics (e.g., mitomycin, adriamycin); plant-derived antitumor agents (e.g., vincristine, vindesine, TAXOL^{®}, paclitaxel, abraxane); cisplatin; carboplatin; etoposide; and the like. Such agents may further include, but are not limited to, the anti-cancer agents trimethotrexate (TMTX); temozolomide; raltitrexed; S-(4-Nitrobenzyl)-6-thioinosine (NBMPR); 6-benzyguanidine (6-BG); a nitrosoureas a nitrosourea (rabinopyranosyl-N-methyl-N-nitrosourea (Aranose), Carmustine (BCNU, BiCNU), Chlorozotocin, Ethylnitrosourea (ENU), Fotemustine, Lomustine (CCNU), Nimustine, N-Nitroso-N-methylurea (NMU), Ranimustine (MCNU), Semustine, Streptozocin (Streptozotocin)); cytarabine; and camptothecin; or a therapeutic derivative of any thereof.

The phrase "therapeutically effective amount" or an "effective amount refers to the administration of an agent to a subject, either alone or as part of a pharmaceutical composition and either in a single dose or as part of a series of doses, in an amount capable of having any detectable, positive effect on any symptom, aspect, or characteristic of a disease, disorder or condition when administered to the subject. The therapeutically effective amount can be ascertained by measuring relevant physiological effects, and it can be adjusted in connection with the dosing regimen and diagnostic analysis of the subject's condition, and the like. By way of example, measurement of the amount of inflammatory cytokines produced following administration can be indicative of whether a therapeutically effective amount has been used. In reference to cancer or pathologies related to unregulated cell division, a therapeutically effective amount refers to that amount which has the effect of (1) reducing the size of a tumor (i.e. tumor regression), (2) inhibiting (that is, slowing to some extent, preferably stopping) aberrant cell division, for example cancer cell division, (3) preventing or reducing the metastasis of cancer cells, and/or, (4) relieving to some extent (or, preferably, eliminating) one or more symptoms associated with a pathology related to or caused in part by unregulated or aberrant cellular division, including for example, cancer. An "effective amount" is also that amount that results in desirable PD and PK profiles and desirable immune cell profiling upon administration of the therapeutically active compositions for use of the invention.

The terms "treating" or "treatment" of a disease (or a condition or a disorder) as used herein refer to preventing the disease from occurring in a human subject or an animal subject that may be predisposed to the disease but does not yet experience or exhibit symptoms of the disease (prophylactic treatment), inhibiting the disease (slowing or arresting its development), providing relief from the symptoms or side-effects of the disease (including palliative treatment), and causing regression of the disease. With regard to cancer, these terms also mean that the life expectancy of an individual affected with a cancer may be increased or that one or more of the symptoms of the disease will be reduced. With regard to cancer, "treating" also includes enhancing or prolonging an anti-tumor response in a subject.

As used herein any form of administration of a "combination", "combined therapy" and/or "combined treatment regimen" refers to at least two therapeutically active drugs or compositions which may be administered simultaneously, in either separate or combined formulations, or sequentially at different times separated by minutes, hours or days, but in some way act together to provide the desired therapeutic response.

The term "enhancing", as used herein, refers to allowing a subject or tumor cell to improve its ability to respond to a treatment disclosed herein. For example, an enhanced response may comprise an increase in responsiveness of at least 5%, 10%, 15%, 20%, 25%, 30%, 35%, 40%, 45%, 50%, 55%, 60%, 65%, 70%, 75%, 80%, 85%, 90%, 95% or 98% or more. As used herein, "enhancing" can also refer to enhancing the number of subjects who respond to a treatment such as a combination therapy comprising chemotherapy, drug-resistant immunocompetent cells, and immune checkpoint inhibitors. For example, an enhanced response may refer to a total percentage of subjects who respond to a treatment wherein the percentage is of at least 5%, 10%, 15%, 20%, 25%, 30%, 35%, 40%, 45%, 50%, 55%, 60%, 65%, 70%, 75%, 80%, 85%, 90%, 95% or 98% or more.

The terms "subject" and "patient" as used herein include humans, mammals (e.g., cats, dogs, horses, etc.), living cells, and other living organisms. A living organism can be as simple as, for example, a single eukaryotic cell or as complex as a mammal. Typical patients are mammals, particularly primates, especially humans. For veterinary applications, a wide variety of subjects will be suitable, e.g., livestock such as cattle, sheep, goats, cows, swine, and the like; poultry such as chickens, ducks, geese, turkeys, and the like; and domesticated animals particularly pets such as dogs and cats. For diagnostic or research applications, a wide variety of mammals will be suitable subjects, including rodents (e.g., mice, rats, hamsters), rabbits, primates, and swine such as inbred pigs and the like. Preferably, a system includes a sample and a subject. The term "living host" refers to host or organisms noted above that are alive and are not dead. The term "living host" refers to the entire host or organism and not just a part excised (e.g., a liver or other organ) from the living host.

The term "γδ T-cells (gamma delta T-cells)" as used herein refers to a small subset of T-cells that express a distinct T-cell receptor (TCR) on their surface. A majority of T-cells have a TCR composed of two glycoprotein chains called α- and β-TCR chains. In contrast, in γδ T-cells, the TCR is made up of one γ-chain and one δ-chain. This group of T-cells is usually much less common than αβ T-cells. γδ T-cells are unique amongst T cell types in that they do not require antigen processing and MHC presentation of peptide epitopes. Furthermore, γδ T-cells are believed to have a prominent role in recognition of lipid antigens, and to respond to stress-related antigens such as MIC-A and MIC-B and other ligands of the NKG2D receptor.

The term "drug resistant immunotherapy" or DRI is a gene-based strategy for treating cancer whereby anti-cancer immune cells, preferably γδ T cells are genetically engineered to resist the toxic effects of chemotherapy drugs which allows for the combined administration of chemotherapy and immunotherapy.

The term "enriched", as used herein, refers to increasing the total percentage of one or more cytotoxic immune cell types present (e.g., γδ T-cells and/or NK cells) 1 in a sample, relative to the total percentage of the same one or more cell types prior to enrichment, as disclosed herein. For example, a sample that is "enriched" for a for one or more types of cytotoxic immune cell may comprise between about 10% to 100% of the one or more cytotoxic immune cell types in the sample, whereas the total percentage of one or more of the cytotoxic immune cell types in a sample prior to enrichment was, for example, between 0% and 10%. Preferably, an enriched sample comprises at least 10%, 15%, 20%, 25%, 30%, 35%, 40%, 50%, 60% ,70%, 80%, 90% or 100%, of one or more types of cytotoxic immune cell. Samples may be enriched for one or more cell types using standard techniques, for example, flow cytometry techniques.

The term "highly enriched", as used herein, refers to increasing the total percentage of one or more cytotoxic immune cell types in a sample such that the one or more cytotoxic immune cell types may comprise between at least about 70% to about 100% of the cytotoxic immune cell type in the sample, whereas the total percentage of that same type of cytotoxic immune cell prior to enrichment was, for example, between 0% and 10%. Preferably, a highly enriched sample comprises at least 60%, 65%, 70%, 75%, 80%, 85%, 90%, 95%, 99% or more of one or more types of cytotoxic immune cell. Samples may be highly enriched for one or more cell types using standard techniques, for example, flow cytometry techniques.

The terms "expanded" and "expansion" as used herein with regard to expansion of one or more cytotoxic immune cells in a sample means to increase in the number of one or more cytotoxic immune cells in a sample by, for example about at least 2-fold, preferably by about 5-fold, preferably by at least 10-fold, preferably about at least 50-fold or more. Expansion of a cytotoxic immune cell population can be accomplished by any number of methods as are known in the art. For example, T-cells can be rapidly expanded using non-specific T-cells receptor stimulation in the presence of feeder lymphocytes and either interleukin-2 (IL-2) or interleukin-15 (IL-15), with IL-2 being preferred. The non-specific T-cells receptor stimulus can include around 30 ng/ml of OKT3, a mouse monoclonal anti-CD3 antibody (available from ORTHO-MCNEIL^{®}, Raritan, N.J.). Alternatively T-cells can be rapidly expanded by stimulation of peripheral blood mononuclear cells (PBMC) *in vitro* with one or more antigens (including antigenic portions thereof, such as epitope(s), or a cell) of the cancer, which can be optionally expressed from a vector, such as an human leukocyte antigen A2 (HLA-A2) binding peptide, e.g., 0.3 µM MART-1:26-35 (27 L) or gp100:209-217 (210M), in the presence of a T-cell growth factor, such as 300 IU/ml IL-2 or IL-15, with IL-2 being preferred.

The terms "isolated' and isolated population of cells" as used herein refers to a cell or a plurality of cells removed from the tissue or state in which they are found in a subject. The terms may further include cells that have been separated according to such parameters as, but not limited to, cell surface markers, a reporter marker such as a dye or label.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from said RNA nucleic acid molecule to give a protein, a polypeptide, or a portion or fragment thereof.

The term "recombinant cell" refers to a cell that has a new combination of nucleic acid segments that are not covalently linked to each other in nature. A new combination of nucleic acid segments can be introduced into an organism using a wide array of nucleic acid manipulation techniques available to those skilled in the art. A recombinant cell can be a single eukaryotic cell, or a single prokaryotic cell, or a mammalian cell. The recombinant cell may harbor a vector that is extragenomic. An extragenomic nucleic acid vector does not insert into the cell's genome. A recombinant cell may further harbor a vector or a portion thereof that is intragenomic. The term "intragenomic" defines a nucleic acid construct incorporated within the recombinant cell's genome.

The terms "recombinant nucleic acid" and "recombinant DNA" as used herein refer to combinations of at least two nucleic acid sequences that are not naturally found in a eukaryotic or prokaryotic cell. The nucleic acid sequences include, but are not limited to, nucleic acid vectors, gene expression regulatory elements, origins of replication, suitable gene sequences that when expressed confer antibiotic resistance, protein-encoding sequences, and the like. The term "recombinant polypeptide" is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

The term "targeted therapy", as used herein, refers to any therapeutic molecule that targets any aspect of the immune system.

The terms "transformation", "transduction" and "transduction" all denote the introduction of a polynucleotide into a recipient cell or cells.

"Immune checkpoint proteins" regulate T cell function in the immune system. T cells play a central role in cell-mediated immunity. Checkpoint proteins interact with specific ligands that send a signal into the T cell and essentially switch off or inhibit T cell function. Cancer cells take advantage of this system by driving high levels of expression of checkpoint proteins on their surface that results in control of the T cells expressing checkpoint proteins on the surface of T cells that enter the tumor microenvironment, thus suppressing the anticancer immune response. As such, inhibition of checkpoint proteins by agents referred to herein as "immune checkpoint protein (ICP) inhibitors" would result in restoration of T cell function and an immune response to the cancer cells. Examples of checkpoint proteins include, but are not limited to: CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, OX40, B-7 family ligands or a combination thereof. Preferably, the immune checkpoint inhibitor interacts with a ligand of a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, OX40, A2aR, B-7 family ligands or a combination thereof. Preferably, the checkpoint inhibitor is a biologic therapeutic or a small molecule. Preferably, the checkpoint inhibitor is a monoclonal antibody, a humanized antibody, a fully human antibody, a fusion protein or a combination thereof. There are several PD-1and CTLA-4 inhibitors currently being tested in clinical trials. CT-011 is a humanized IgG1 monoclonal antibody against PD-1. BMS 936558 is a fully human IgG4 monoclonal antibody targeting PD-1 agents. BMS 936559 is a fully human IgG4 monoclonal antibody that targets the PD-1 ligand PD-L1. MK 3475 is a humanized IgG4 anti-PD-1 monoclonal antibody in phase I development in a five-part study evaluating the dosing, safety, and tolerability of the drug in subjects with progressive, locally advanced, or metastatic carcinoma, melanoma, or NSCLC. MPDL 3280A is a monoclonal antibody. AMP 224 is a fusion protein of the extracellular domain of the second PD-1 ligand, PD-L2, and IgG1, which has the potential to block the PD-L2/PD-1 interaction. Medi 4736 is an anti-PD-L1 antibody in phase I clinical testing in subjects with advanced malignant melanoma, renal cell carcinoma, NSCLC, and colorectal cancer. A first-in-class immunotherapy, ipilimumab (YERVOY^{®}), a monoclonal antibody that targets CTLA-4 on the surface of T cells, was for the treatment of melanoma.

### γδ T Cell Immunotherapy Combinations

The disclosure provides combination therapies for reducing tumors and treating cancer in a patient comprising compositions and methods for γδ T cell immunotherapy in combination DDR inhibitors, including but not limited to PARP inhibitors and in further combination with chemotherapeutic agents including but not limited to temozolomide (TMZ). Preferably, the combination of γδ T cell immunotherapy and PARP inhibitors for the treatment of cancer further includes combinations with other immunotherapies such as immune checkpoint (ICP) blockade therapy and/or other immunostimulatory agents.

The disclosure provides methods for treating cancer in a patient, comprising, i) administering a composition comprising an optionally enriched and/or optionally expanded population of γδ T cells; ii) administering to a patient in need thereof the composition of (i); administering to the patient an effective amount of at least one DDR inhibitor; and iii) optionally administering a chemotherapeutic agent, thereby treating cancer in the patient.

Preferably, γδ T-cells derived from human induced pluripotent stem cells (hiPSCs). Preferably, the pluripotent stem cells can be isolated from the patient having the cancer. Preferably, the pluripotent stem cells may be isolated from a source other than the patient with cancer. Preferably, the optionally enriched and/or optionally expanded compositions comprising γδ T-cells also comprise natural killer (NK) cells and optionally further comprise other immunocompetent cells including but not limited to: monocytes, macrophages and dendritic cells.

Preferably compositions comprising optionally enriched and/or optionally expanded population of γδ T cells comprise at least about 50%, at least about 60%, at least about 70% or more of γδ T cells. Preferably, compositions comprising optionally enriched and/or optionally expanded population of γδ T cells comprise less than about 35% natural killer (NK) cells. Preferably, compositions comprising optionally enriched and/or optionally expanded population of γδ T cells comprise less than about 10%, less than about 5% αβ T cells.

Preferably, therapeutic compositions for administration to a patient comprising optionally enriched and/or optionally expanded population of γδ T cells comprise about 5x10⁸ γδ T cells/kg or less of a patient's weight. Preferably, therapeutic compositions for administration to a patient comprising optionally enriched and/or optionally expanded population of γδ T cells comprise about 5x10⁷ γδ T cells/kg or less of a patient's weight. Preferably, therapeutic compositions for administration to a patient comprising optionally enriched and/or optionally expanded population of γδ T cells comprise about 5x10⁶ γδ T cells/kg or less of a patient's weight.

Methods for isolating γδ T-cells either from a patient to be treated or from another source, as described, for example, by Lamb L. S. in U.S. Pat. No. 7,078,034.

A DDR inhibitor suitable for use as described herein may be any compound or entity, such as a small organic molecule, peptide or nucleic acid, which inhibits, reduces or abolishes the activity of one or more components of a DNA damage repair pathway. These pathways include base excision repair (BER), homologous recombination (HR) dependent DNA double strand break (DSB) repair, non-homologous end joining (NHEJ), nucleotide excision repair (NER), base excision repair (BER) and mismatch repair (MMR).

The DDR inhibitor reduces or abolishes the activity of the enzyme poly (ADP-ribose) polymerase (PARP). Preferred PARP inhibitors include, but are not limited to: niraparib, olaparib, and rucaparib all approved by the FDA. Olaparib and rucaparib, have been approved by the FDA for the treatment of recurrent, BRCA-associated ovarian cancer. More recently, these two and a third PARP inhibitor, niraparib, were approved by the FDA as maintenance therapy following platinum-based chemotherapy for recurrent ovarian cancer.

Other preferred PARP inhibitors include but are not limited to: Talazoparib (Pfizer), veliparib (Abbvie), E7016 (Eisai), CEP-9722 (Teva), and BGB-290 (Pamiparib, BeiGene).

Other examples of compounds which are known PARP inhibitors include:
1. Nicotinamides, such as 5-methyl nicotinamide and O-(2-hydroxy-3-piperidino-propyl)-3-carboxylic acid amidoxime, and analogues and derivatives thereof;
2. Benzamides, including 3-substituted benzamides such as 3-aminobenzamide, 3-hydroxybenzamide 3-nitrosobenzamide, 3-methoxybenzamide and 3-chloroprocainamide, and 4-aminobenzamide, 1,5-di[(3-carbamoylphenyl)aminocarbonyloxy]pentane, and analogues and derivatives thereof;
3. Isoquinolinones and Dihydroisoquinolinones, including 2H-isoquinolin-1-ones, 3H-quinazolin-4-ones, 5-substituted dihydroisoquinolinones such as 5-hydroxy dihydroisoquinolinone, 5-methyl dihydroisoquinolinone, and 5-hydroxy isoquinolinone, 5-amino isoquinolin-1-one, 5-dihydroxyisoquinolinone, 3,4 dihydroisoquinolin-1(2H)-ones such as 3,4 dihydro-5-methoxy-isoquinolin-1(2H)-one and 3,4 dihydro-5-methyl-1(2H)isoquinolinone, isoquinolin-1(2H)-ones, 4,5-dihydro-imidazo[4,5,1-ij]quinolin-6-ones, 1,6,-naphthyridine-5(6H)-ones, 1,8-naphthalimides such as 4-amino-1,8-naphthalimide, isoquinolinone, 3,4-dihydro-5-[4-1(1-piperidinyl) butoxy]-1(2H)-isoquinolinone, 2,3-dihydrobenzo[de]isoquinolin-1-one, 1-11b-dihydro-[2H]benzopyrano[4,3,2-de]isoquinolin-3-one, and tetracyclic lactams, including benzpyranoisoquinolinones such as benzopyrano[4,3,2-de] isoquinolinone, and analogues and derivatives thereof;
4. Benzimidazoles and indoles, including benzoxazole-4-carboxamides, benzimidazole-4-carboxamides, such as 2-substituted benzoxazole 4-carboxamides and 2-substituted benzimidazole 4-carboxamides such as 2-aryl benzimidazole 4-carboxamides and 2-cycloalkylbenzimidazole-4-carboxamides including 2-(4-hydroxphenyl) benzimidazole 4-carboxamide, quinoxalinecarboxamides, imidazopyridinecarboxamides, 2-phenylindoles, 2-substituted benzoxazoles, such as 2-phenyl benzoxazole and 2-(3-methoxyphenyl) benzoxazole, 2-substituted benzimidazoles, such as 2-phenyl benzimidazole and 2-(3-methoxyphenyl) benzimidazole, 1,3,4,5 tetrahydro-azepino[5,4,3-cd]indol-6-one, azepinoindoles and azepinoindolones such as 1,5 dihydro-azepino[4,5,6-cd]indolin-6-one and dihydrodiazapinoindolinone, 3-substituted dihydrodiazapinoindolinones such as 3-(4-trifluoromethylphenyl)-dihydrodiazapinoindolinone, tetrahydrodiazapinoindolinone and 5,6,-dihydroimidazo[4,5,1-j, k][1,4]benzodiazopin-7(4H)-one, 2-phenyl-5,6-dihydro-imidazo[4,5,1-jk][1,4]benzodiazepin-7(4H)-one and 2,3, dihydro-isoindol-1-one, and analogues and derivatives thereof;
5. Phthalazin-1(2H)-ones and quinazolinones, such as 4-hydroxyquinazoline, phthalazinone, 5-methoxy-4-methyl-1(2) phthalazinones, 4-substituted phthalazinones, 4-(1-piperazinyl)-1(2H)-phthalazinone, tetracyclic benzopyrano[4,3,2-de]phthalazinones and tetracyclic indeno[1,2,3-de]phthalazinones and 2-substituted quinazolines, such as 8-hydroxy-2-methylquinazolin-4-(3H) one, tricyclic phthalazinones and 2-aminophthalhydrazide, and analogues and derivatives thereof;
6. Isoindolinones and analogues and derivatives thereof;
7. Phenanthridines and phenanthridinones, such as 5[H]phenanthridin-6-one, substituted 5[H]phenanthridin-6-ones, especially 2-, 3-substituted 5[H]phenanthridin-6-ones and sulfonamide/carbamide derivatives of 6(5H)phenanthridinones, thieno[2,3-c]isoquinolones such as 9-amino thieno[2,3-c]isoquinolone and 9-hydroxythieno[2,3-c]isoquinolone, 9-methoxythieno[2,3-c]isoquinolone, and N-(6-oxo-5,6-dihydrophenanthridin-2-yl]-2-(N,N-dimethylamino}acetamide, substituted 4,9-dihydrocyclopenta[lmn]phenanthridine-5-ones, and analogues and derivatives thereof;
8. Benzopyrones such as 1,2-benzopyrone 6-nitrosobenzopyrone, 6-nitroso 1,2-benzopyrone, and 5-iodo-6-aminobenzopyrone, and analogues and derivatives thereof;
9. Unsaturated hydroximic acid derivatives such as O-(3-piperidino-2-hydroxy-1-propyl)nicotinic amidoxime, and analogues and derivatives thereof;
10. Pyridazines, including fused pyridazines and analogues and derivatives thereof; and
11. Other compounds such as caffeine, theophylline, and thymidine, and analogues and derivatives thereof.

### Combination of γδ T cell, DRI and PARP Inhibitor Therapy

Preferably, the combination of γδ T cell immunotherapy and PARP inhibitor therapy as a treatment regimen further includes combinations with chemotherapeutic agents, wherein the γδ T cells are genetically modified to impart resistance to the chemotherapeutic agents known as drug resistant immunotherapy (DRI). Preferably and genetic modification of the γδ T-cells to make them resistant to the effects of the chemotherapeutic agents does not affect the γδ T-cells' ability to kill target cancer cells in the presence or absence of a chemotherapy agent. The γδ T-cells are genetically modified using standard recombinant techniques such as those described in the Example, and in WO 2011/053750 and Lamb et al., PloS ONE 8(1): e51805. doi:10.1371/journal.pone.0051805).

Preferably compositions comprising optionally enriched and/or optionally expanded population of genetically engineered γδ T cells comprise at least about 50%, at least about 60%, at least about 70% or more of γδ T cells. Preferably, compositions comprising optionally enriched and/or optionally expanded genetically engineered population of γδ T cells comprise less than about 35% natural killer (NK) cells. Preferably, compositions comprising optionally enriched and/or optionally expanded population of genetically engineered γδ T cells comprise less than about 10%, less than about 5% αβ T cells.

Preferably, therapeutic compositions for administration to a patient comprising optionally enriched and/or optionally expanded population of genetically engineered γδ T cells comprise about 5x10⁸ γδ T cells/kg or less of a patient's weight. Preferably, therapeutic compositions for administration to a patient comprising optionally enriched and/or optionally expanded population of genetically engineered γδ T cells comprise about 5x10⁷ γδ T cells/kg or less of a patient's weight. Preferably, therapeutic compositions for administration to a patient comprising optionally enriched and/or optionally expanded population of genetically engineered γδ T cells comprise about 5x10⁶ γδ T cells/kg or less of a patient's weight.

A major limitation to chemotherapy treatments for cancer is drug induced immune toxicity which causes the killing of immunocompetent cells and loss of an effective immune system that would otherwise ward off undesirable infections or provide a defense against cancer cells. One strategy to combat the severe toxic effects of chemotherapy would be to selectively genetically modify cytotoxic immune cells by the introduction of retroviral vectors designed to express cDNA sequences that confer drug resistance, which can actively target those cancer cells able to resist the simultaneous administration of a chemotherapeutic agent. Such drug resistant immunotherapy using genetically engineered γδ T cell immunotherapy is described, for example, by Spencer H.T. et al. in US 2015/0017137 (see also, WO 2011/053750 and Lamb et al., PloS ONE 8(1): e51805. doi: 10.1371/journal.pone.0051805).

The inventors have previously discovered that administration of a chemotherapeutic agent such as TMZ transiently increases stress associated antigens (e.g. receptors for the NKG2D family of ligands such as MIC A/B and UL 16 Binding proteins (ULBPs) in TMZ-resistant cell lines. This invention enhances this prior discovery by combining a chemotherapeutic agent with a DDR inhibitor. Without being limited to any theory, the DDR inhibitor such as a PARP inhibitor acts to enhance and prolong the upregulation of stress associated antigens on cancer cells caused by exposure to chemotherapeutic agents. Therefore, tumor cells are made to be highly vulnerable to killing by γδ T cells due to the enhanced forced expression of NKG2D ligands on cancer cells by the combination of chemotherapy and DDR inhibitor therapy for a prolonged period. The modification of the γδ T cells to resist destruction by chemotherapeutic agents enables them to persist in the presence of the chemotherapeutic agent during the optimal and prolonged window of transient NKG2D upregulation by the combined chemotherapeutic agent and DDR inhibitor.

Preferably, the PARP inhibitor is administered to a patient about 1 to about 21 days prior to administration of the chemotherapeutic agent. Preferably the PARP inhibitor is administered about 1 to about 14 days prior to administration of the chemotherapeutic agent. Preferably the PARP inhibitor is administered about 1 to about 7 days prior to administration of the chemotherapeutic agent.

Preferably DRI with the genetically engineered chemotherapy resistant γδ T-cells is administered about 8 to about 72 hours after the administration of the chemotherapeutic agent. Preferably DRI with the genetically engineered chemotherapy resistant γδ T-cells is administered about 8 to about 36 hours after the administration of the chemotherapeutic agent. Preferably DRI with the genetically engineered chemotherapy resistant γδ T-cells is administered about 8 to about 24 hours after the administration of the chemotherapeutic agent. Preferably DRI with the genetically engineered chemotherapy resistant γδ T-cells is administered about 8 to about 12 hours after the administration of the chemotherapeutic agent. Preferably DRI with the genetically engineered chemotherapy resistant γδ T-cells is administered about 12 to about 36 hours after the administration of the chemotherapeutic agent. Preferably DRI with the genetically engineered chemotherapy resistant γδ T-cells is administered about 12 to about 24 hours after the administration of the chemotherapeutic agent. Preferably DRI with the genetically engineered chemotherapy resistant γδ T-cells is administered about 24 hours after the administration of the chemotherapeutic agent. Preferably DRI with the genetically engineered chemotherapy resistant γδ T-cells is administered about 8, 9, 10, 11, 12, 13, 14, 15, 16, 17, 18, 19, 20, 21, 22, 23, 24, 25, 26, 27, 28, 29, 30, 31, 32, 33, 34, 35, or 36 hours after the administration of the chemotherapeutic agent.

### Combination DRI and PARP Inhibitor Therapy with Checkpoint Inhibitors (ICP)

Preferably, the combination of γδ T cell DRI and PARP inhibitor therapy as a treatment regimen further includes combinations with ICP inhibitors. Preferably, the checkpoint inhibitor is a PD-1 inhibitor or a CTLA-4 inhibitor. Preferably, the immune checkpoint inhibitor can be a biologic therapeutic or a small molecule. Preferably, the checkpoint inhibitor is a monoclonal antibody, a humanized antibody, a fully human antibody, a fusion protein, an antigen-binding fragment or a combination thereof.

Preferably, immunostimulatory agents, T cell growth factors and interleukins may be used in the various combination therapies of the invention. Immunostimulatory agents are substances (drugs and nutrients) that stimulate the immune system by inducing activation or increasing activity of any of its components. Immunostimulants include, but are not limited to, bacterial vaccines, colony stimulating factors, interferons, interleukins, other immunostimulants, therapeutic vaccines, vaccine combinations and viral vaccines. T cell growth factors are proteins that stimulate the proliferation of T cells. Examples of T cell growth factors include Il-2, IL-7, IL-15, IL-17, IL21 and IL-33.

Combination therapies comprising drug resistant γδ T cell immunotherapy, chemotherapeutic agents, ICP inhibitors optional immunostimulatory agents are described in WIPO Patent Application WO/2018/035413. Clinical Trails combining PARP inhibitors with immune checkpoint inhibitors are ongoing and have been reported in the literature.

Preferably, combination therapies comprising drug resistant γδ T cell immunotherapy and PARP inhibitors for the treatment of cancer further include immune checkpoint (ICP) blockade therapy and/or therapy with chemotherapeutic agents and/or immunostimulatory agents. may be administered simultaneously, in either separate or combined formulations, or sequentially at different times separated by minutes, hours or days, but in some way act together to provide the desired therapeutic response. The optimal timing of γδ T cell immunotherapy in combination with DDR inhibition and optionally further in combination with chemotherapy and ICP inhibitors will vary across disease settings as is known in the art but will generally see PARP inhibition and/or chemotherapy prior to the administration of a cellular therapy to induce DNA damage and increased immunogenicity.

Preferably, an effective amount of a check point inhibitor is that amount effective to cause tumor regression in combination with DRI and DDR combination therapy. Preferably, the checkpoint inhibitor is administered (in terms of kg of patient body weight) in less than 0.0001 mg/kg, 0.0001-0.001 mg/kg, 0.001-0.01 mg/kg, 0.01-0.05 mg/kg, 0.05-0.1 mg/kg, 0.1-0.2 mg/kg, 0.2-0.3 mg/kg, 0.3-0.5 mg/kg, 0.5-0.7 mg/kg, 0.7-1 mg/kg, 1-2 mg/kg, 2-3 mg/kg, 3-4 mg/kg, 4-5 mg/kg, 5-6 mg/kg, 6-7 mg/kg, 7-8 mg/kg, 8-9 mg/kg, 9-10 mg/kg, at least 10 mg/kg, or any combination thereof doses. Preferably, the checkpoint inhibitor is administered at least once a week, at least twice a week, at least three times a week, at least once every two weeks, or at least once every month or multiple months. Preferably, the checkpoint inhibitor is administered as a single dose, in two doses, in three doses, in four doses, in five doses, or in 6 or more

### Pharmaceutical Compositions and Methods of Administration

Preferably, the combination therapies disclosed herein may be administered to patient by various routes including, for example, orally or parenterally and can include but not be limited to, intravenously, intramuscularly, subcutaneously, intraorbitally, intracapsularly, intraperitoneally, intrarectally, intracisternally, intratumorally, intravasally, intradermally, intravaginally (e.g., vaginal suppositories), or topically (e.g., powders, ointments transdermal patch) or by passive or facilitated absorption through the skin using, for example, a skin patch or transdermal iontophoresis, respectively.

The therapeutic, checkpoint inhibitor, biologic therapeutic or pharmaceutical composition also can be administered to the site of a pathologic condition, for example, intravenously or intra-arterially into a blood vessel supplying a tumor. For example, compositions comprising γδ T-cells, PARP inhibitors or any combination thereof, may be delivered to the targeted tumor directly by such as, but not limited to, direct injection into the tumor mass, delivery to a blood vessel entering the tumor mass, or a combination of both. For example, it is contemplated that the compositions may be delivered to a glioblastoma mass in the brain of a patient by direct implantation through a cannulated needle or catheter inserted into the tumor mass, intracavity post-resection, intraperitoneal or intraventricular.

Preferably, the total amount of an agent to be administered in practicing the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary.

The pharmaceutical compositions of the invention can be formulated to be compatible with the intended method or route of administration; exemplary routes of administration are set forth herein. Furthermore, the pharmaceutical compositions can be used in combination with other therapeutically active agents or compounds as described herein in order to treat or prevent the diseases, disorders and conditions as contemplated by the present disclosure.

The pharmaceutical compositions typically comprise a therapeutically effective amount of one or more agents used in the combination therapies of the invention and one or more pharmaceutically and physiologically acceptable formulation agents. Suitable pharmaceutically acceptable or physiologically acceptable diluents, carriers or excipients include, but are not limited to, antioxidants (e.g., ascorbic acid and sodium bisulfate), preservatives (e.g., benzyl alcohol, methyl parabens, ethyl or n-propyl, p-hydroxybenzoate), emulsifying agents, suspending agents, dispersing agents, solvents, fillers, bulking agents, detergents, buffers, vehicles, diluents, and/or adjuvants. For example, a suitable vehicle can be physiological saline solution or citrate buffered saline, possibly supplemented with other materials common in pharmaceutical compositions for parenteral administration. Neutral buffered saline or saline mixed with serum albumin are further exemplary vehicles. Those skilled in the art will readily recognize a variety of buffers that can be used in the pharmaceutical compositions and dosage forms contemplated herein. Typical buffers include, but are not limited to, pharmaceutically acceptable weak acids, weak bases, or mixtures thereof. As an example, the buffer components can be water soluble materials such as phosphoric acid, tartaric acids, lactic acid, succinic acid, citric acid, acetic acid, ascorbic acid, aspartic acid, glutamic acid, and salts thereof. Acceptable buffering agents include, for example, a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), and N-tris[Hydroxymethyl]methyl-3-aminopropanesulfonic acid (TAPS).

After a pharmaceutical composition has been formulated, it can be stored in sterile vials as a solution, suspension, gel, emulsion, solid, or dehydrated or lyophilized powder. Such formulations can be stored either in a ready-to-use form, a lyophilized form requiring reconstitution prior to use, a liquid form requiring dilution prior to use, or other acceptable form. Preferably, the pharmaceutical composition is provided in a single-use container (e.g., a single-use vial, ampoule, syringe, or autoinjector (similar to, e.g., an EpiPen^{®})), whereas a multi-use container (e.g., a multi-use vial) is provided in other embodiments. Any drug delivery apparatus can be used to deliver IL-10, including implants (e.g., implantable pumps) and catheter systems, slow injection pumps and devices, all of which are well known to the skilled artisan. Depot injections, which are generally administered subcutaneously or intramuscularly, can also be utilized to release the polypeptides disclosed herein over a defined period of time. Depot injections are usually either solid- or oil-based and generally comprise at least one of the formulation components set forth herein. One of ordinary skill in the art is familiar with possible formulations and uses of depot injections.

The pharmaceutical compositions can be in the form of a sterile injectable aqueous or oleagenous suspension. This suspension can be formulated according to the known art using those suitable dispersing or wetting agents and suspending agents mentioned herein. The sterile injectable preparation can also be a sterile injectable solution or suspension in a non-toxic parenterally-acceptable diluent or solvent, for example, as a solution in 1,3-butane diol. Acceptable diluents, solvents and dispersion media that can be employed include water, Ringer's solution, isotonic sodium chloride solution, CREMOPHOR EL^{™} (BASF, Parsippany, N.J.) or phosphate buffered saline (PBS), ethanol, polyol (e.g., glycerol, propylene glycol, and liquid polyethylene glycol), and suitable mixtures thereof. In addition, sterile, fixed oils are conventionally employed as a solvent or suspending medium. For this purpose, any bland fixed oil can be employed, including synthetic mono- or diglycerides. Moreover, fatty acids such as oleic acid, find use in the preparation of injectables. Prolonged absorption of particular injectable formulations can be achieved by including an agent that delays absorption (e.g., aluminum monostearate or gelatin).

The pharmaceutical compositions can be in a form suitable for oral use, for example, as tablets, capsules, troches, lozenges, aqueous or oily suspensions, dispersible powders or granules, emulsions, hard or soft capsules, or syrups, solutions, microbeads or elixirs. Pharmaceutical compositions intended for oral use can be prepared according to any method known to the art for the manufacture of pharmaceutical compositions, and such compositions can contain one or more agents such as, for example, sweetening agents, flavoring agents, coloring agents and preserving agents in order to provide pharmaceutically elegant and palatable preparations. Tablets, capsules and the like contain the active ingredient in admixture with non-toxic pharmaceutically acceptable excipients which are suitable for the manufacture of tablets. These excipients can be, for example, diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc.

The tablets, capsules and the like suitable for oral administration can be uncoated or coated by known techniques to delay disintegration and absorption in the gastrointestinal tract and thereby provide a sustained action. For example, a time-delay material such as glyceryl monostearate or glyceryl distearate can be employed. They can also be coated by techniques known in the art to form osmotic therapeutic tablets for controlled release. Additional agents include biodegradable or biocompatible particles or a polymeric substance such as polyesters, polyamine acids, hydrogel, polyvinyl pyrrolidone, polyanhydrides, polyglycolic acid, ethylene-vinylacetate, methylcellulose, carboxymethylcellulose, protamine sulfate, or lactide/glycolide copolymers, polylactide/glycolide copolymers, or ethylenevinylacetate copolymers in order to control delivery of an administered composition. For example, the oral agent can be entrapped in microcapsules prepared by coacervation techniques or by interfacial polymerization, by the use of hydroxymethylcellulose or gelatin-microcapsules or poly (methylmethacrolate) microcapsules, respectively, or in a colloid drug delivery system. Colloidal dispersion systems include macromolecule complexes, nano-capsules, microspheres, microbeads, and lipid-based systems, including oil-in-water emulsions, micelles, mixed micelles, and liposomes. Methods for the preparation of the above-mentioned formulations will be apparent to those skilled in the art.

Formulations for oral use can also be presented as hard gelatin capsules wherein the active ingredient is mixed with an inert solid diluent, for example, calcium carbonate, calcium phosphate, kaolin or microcrystalline cellulose, or as soft gelatin capsules wherein the active ingredient is mixed with water or an oil medium, for example peanut oil, liquid paraffin, or olive oil.

Aqueous suspensions contain the active materials in admixture with excipients suitable for the manufacture thereof. Such excipients can be suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents, for example a naturally-occurring phosphatide (e.g., lecithin), or condensation products of an alkylene oxide with fatty acids (e.g., polyoxy-ethylene stearate), or condensation products of ethylene oxide with long chain aliphatic alcohols (e.g., for heptadecaethyleneoxycetanol), or condensation products of ethylene oxide with partial esters derived from fatty acids and a hexitol (e.g., polyoxyethylene sorbitol monooleate), or condensation products of ethylene oxide with partial esters derived from fatty acids and hexitol anhydrides (e.g., polyethylene sorbitan monooleate). The aqueous suspensions can also contain one or more preservatives.

Oily suspensions can be formulated by suspending the active ingredient in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents such as those set forth above, and flavoring agents can be added to provide a palatable oral preparation.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified herein.

The pharmaceutical compositions can also be in the form of oil-in-water emulsions. The oily phase can be a vegetable oil, for example olive oil or arachis oil, or a mineral oil, for example, liquid paraffin, or mixtures of these. Suitable emulsifying agents can be naturally occurring gums, for example, gum acacia or gum tragacanth; naturally occurring phosphatides, for example, soy bean, lecithin, and esters or partial esters derived from fatty acids; hexitol anhydrides, for example, sorbitan monooleate; and condensation products of partial esters with ethylene oxide, for example, polyoxyethylene sorbitan monooleate.

Formulations can also include carriers to protect the composition against rapid degradation or elimination from the body, such as a controlled release formulation, including implants, liposomes, hydrogels, prodrugs and microencapsulated delivery systems. For example, a time delay material such as glyceryl monostearate or glyceryl stearate alone, or in combination with a wax, can be employed.

Suppositories can be prepared by mixing the drug with a suitable non-irritating excipient which is solid at ordinary temperatures but liquid at the rectal temperature and will therefore melt in the rectum to release the drug. Such materials include, but are not limited to, cocoa butter and polyethylene glycols.

The pharmaceutical compositions suitable for use in accordance with the invention may be in any format (e.g., sprays for nasal or inhalation use) currently known or developed in the future.

### Treatment Indications

The combination treatment methods described herein are particularly suitable for the treatment of cancer. Cancer cells can invade nearby tissues and can spread through the bloodstream and lymphatic system to other parts of the body. There are several main types of cancer, for example, carcinoma is cancer that begins in the skin or in tissues that line or cover internal organs. Sarcoma is cancer that begins in bone, cartilage, fat, muscle, blood vessels, or other connective or supportive tissue. Leukemia is cancer that starts in blood-forming tissue such as the bone marrow and causes large numbers of abnormal blood cells to be produced and enter the bloodstream. Lymphoma is cancer that begins in the cells of the immune system.

When normal cells lose their ability to behave as a specified, controlled and coordinated unit, a tumor is formed. Generally, a solid tumor is an abnormal mass of tissue that usually does not contain cysts or liquid areas (some brain tumors do have cysts and central necrotic areas filled with liquid). A single tumor may even have different populations of cells within it, with differing processes that have gone awry. Solid tumors may be benign (not cancerous), or malignant (cancerous). Different types of solid tumors are named for the type of cells that form them. Examples of solid tumors are sarcomas, carcinomas, and lymphomas. Leukemias (cancers of the blood) generally do not form solid tumors.

A tumor can be classified as malignant or benign. In both cases, there is an abnormal aggregation and proliferation of cells. In the case of a malignant tumor, these cells behave more aggressively, acquiring properties of increased invasiveness. Ultimately, the tumor cells may even gain the ability to break away from the microscopic environment in which they originated, spread to another area of the body (with a very different environment, not normally conducive to their growth), and continue their rapid growth and division in this new location. This is called metastasis. Once malignant cells have metastasized, achieving a cure is more difficult. Benign tumors have less of a tendency to invade and are less likely to metastasize.

Examples of the solid tumor cancers that may be treated using the combination treatment regimens described herein include, but are not limited to: pancreatic cancer, colorectal cancer, non-small cell lung cancer, renal cell carcinoma; squamous cell carcinoma of the head and neck, bladder cancer, cancers of the prostate, cervix, stomach, endometrium, brain, liver, ovary, testis, head, neck, skin (including melanoma and basal carcinoma), mesothelial lining, esophagus, breast, muscle, connective tissue, lung (including small-cell lung carcinoma and non-small-cell carcinoma), adrenal gland, thyroid, kidney, or bone; glioblastoma, mesothelioma, gastric cancer, sarcoma, choriocarcinoma, cutaneous basocellular carcinoma, and testicular seminoma. In preferred aspects, the cancer is cervical cancer, non-small cell lung cancer, renal cell carcinoma; squamous cell carcinoma of the head and neck, bladder cancer, pancreatic cancer, melanoma, lymphoma or gastric cancer. In more preferred aspects, the cancer is melanoma, non-small cell lung cancer, squamous cell carcinoma of the head and neck, bladder cancer, renal cell carcinoma or gastric carcinoma. The treatment regimens of the invention are particularly suited for treating solid tumors including but not limited to: lymphomas, melanoma, renal cell carcinoma (RCC), advanced solid tumors, tumors that have previously been treated with therapeutic therapy but remain refractory to previous therapies.

Cancers that may also be treated in accordance with invention include, but are not limited to, Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Glioblastoma, Childhood; Glioblastoma, Adult; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland' Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor, among others.

Preferably, the cancer being treated in accordance with the invention is a CNS tumor including, but not limited to, intracranial and spinal ependymoma (excluding subependymoma); low grade infiltrative supratentorial astrocytoma/oligodendroglioma, medulloblastoma, anaplastic gliomas, glioblastoma, metastatic lesion of the CNS and primary CNS lymphoma.

Preferably, the cancer being treated is a melanoma. Preferably the cancer being treated is uveal melanoma.

Preferably, the cancer being treated is a neuroendocrine or adrenal tumor. Examples include but are not limited to bronchopulmonary disease, GI tract, lung or thymus, pancreas, paraganglioma or pheochromocytoma.

Preferably, the cancer being treated is non-Hodgkin's lymphoma including but not limited to mycosis fungoides and Sezary syndrome.

Preferably the cancer being treated is a soft tissue sarcoma. Examples include angiosarcoma, unresectable or progressive retroperitoneal/intra-abdominal soft tissue sarcoma, rhabdomyosarcoma, extremity/superficial trunk and/or head and neck cancer, or solitary fibrous tumor/hemangiopericytoma.

Preferably the cancer being treated is bone cancer. Examples include Ewing's sarcoma and mesenchymal chondrosarcoma.

Preferably, the cancer being treated is uterine sarcoma, small cell lung cancer (SCLC) or Zollinger-Ellison syndrome.

Preferably, the cancer being treated in accordance with the invention is a gynecologic cancer (e.g., cancers of the female reproductive system) including, but not limited to: ovarian cancer, cancer of the fallopian tube(s), peritoneal cancer and breast cancer. Preferably the cancer being treated in accordance with the invention is ovarian cancer.

Preferably, a cancer being treated in accordance with the invention is glioblastoma.

Brain tumors spread extensively within the brain but do not usually metastasize outside the brain. Gliomas are very invasive inside the brain, even crossing hemispheres. They do divide in an uncontrolled manner, though. Depending on their location, they can be just as life threatening as malignant lesions. An example of this would be a benign tumor in the brain, which can grow and occupy space within the skull, leading to increased pressure on the brain.

### Kits

Also provided are kits comprising the pharmaceutical compositions typically comprise a therapeutically effective amount of one or more agents used in the combination therapies disclosed described herein. Kits typically include a label indicated the intended use of the contents of the kits and instructions for use.

The kits are generally in the form of a physical structure housing various components, as described below, and can be utilized, for example, in practicing the methods described above. A kit can include a composition comprising one or more of the therapeutic agents used in the combination therapy of the disclosure (e.g. genetically engineered γδ cells or a DRI inhibitor or ICP inhibitor) provided in, e.g., one or more sterile containers, which can be in the form of a pharmaceutical composition suitable for administration to a subject. The pharmaceutical composition can be provided in a form that is ready for use or in a form requiring, for example, reconstitution or dilution prior to administration. When they compositions are in a form that needs to be reconstituted by a user, the kit can also include buffers, pharmaceutically acceptable excipients, and the like, packaged with or separately the therapeutic agent. When combination therapy is contemplated, the kit can contain the several agents separately or they can already be combined in the kit.

A kit of the disclosure can be designed for conditions necessary to properly maintain the components housed therein (e.g., refrigeration or freezing). A kit can contain a label or packaging insert including identifying information for the components therein and instructions for their use (e.g., dosing parameters, clinical pharmacology of the active ingredient(s), including mechanism(s) of action, pharmacokinetics and pharmacodynamics, adverse effects, contraindications, etc.).

Each component of the kit can be enclosed within an individual container, and all of the various containers can be within a single package. Labels or inserts can include manufacturer information such as lot numbers and expiration dates. The label or packaging insert can be, e.g., integrated into the physical structure housing the components, contained separately within the physical structure, or affixed to a component of the kit (e.g., an ampule, syringe or vial).

Labels or inserts can additionally include, or be incorporated into, a computer readable medium, such as a disk (e.g., hard disk, card, memory disk), optical disk such as CD- or DVD-ROM/RAM, DVD, MP3, magnetic tape, or an electrical storage media such as RAM and ROM or hybrids of these such as magnetic/optical storage media, FLASH media or memory-type cards. In some embodiments, the actual instructions are not present in the kit, but means for obtaining the instructions from a remote source, e.g., via an internet site, are provided.

### EXAMPLES

The following examples are offered by way of illustration and are not to be construed as limiting the invention as claimed in any way.

### Example 1- Cancer Treatment Paradigms

A combination therapy comprising a chemotherapeutic agent, drug-resistant yδ-T cells, a PARP-1 inhibitor, and an immune checkpoint inhibitor(s) is used to treat the cancer patient. For the treatment of ovarian cancer, niraparib, olaparib, or rucaparib, are used as the PARP inhibitor. For the treatment of glioblastoma, olaparib or niraparib are used as the PARP inhibitor. The chemotherapeutic agent is selected from alkylating agents (e.g., cyclophosphamide, ifosfamide), metabolic antagonists (e.g., methotrexate (MTX), 5-fluorouracil or derivatives thereof), antitumor antibiotics (e.g., mitomycin, adriamycin), plant-derived antitumor agents (e.g., vincristine, vindesine, TAXOL^{®}, paclitaxel, abraxane), cisplatin, carboplatin, etoposide, and the like. Such agents may further include, but are not limited to, the anti-cancer agents trimethotrexate (TMTX), temozolomide, raltitrexed, S-(4-Nitrobenzyl)-6-thioinosine (NBMPR), 6-benzyguanidine (6-BG), bis-chloronitrosourea (BCNU), cytarabine, and camptothecin, or a therapeutic derivative of any thereof. The drug-resistant yδ-T cells are resistant to the chemotherapeutic agent. The immune checkpoint inhibitor(s) is used alone or in combination with other immune checkpoint inhibitors, and is selected from inhibitors of the immune checkpoint proteins CTLA-4, PDL1 (B7-H1, CD274), PDL2 (B7-DC, CD273), PD1, B7-H3 (CD276), B7-H4 (B7-S1, B7x, VCTN1), BTLA (CD272), HVEM, TIM3 (HAVcr2), GAL9, LAG3 (CD223), VISTA, KIR, 2B4 (CD244; belongs to the CD2 family of molecules and is expressed on all NK, γδ, and memory CD8⁺ (αβ) T cells), CD160 (also referred to as BY55), CGEN-15049, CHK 1 and CHK2 kinases, OX40, A2aR and various B-7 family ligands.

### Evaluation of Immune Therapy Activity in Combination with Chemotherapy:

Response assessment criteria include evaluation based upon Response Evaluation Criteria in Solid Tumors (WHO; World Health Organization Offset Publication No. 48, 1979); RECIST (Response Evaluation Criteria in Solid Tumors; J Natl Cancer Inst 2000; 92:205-16; Eisenhauer EA et al., Eur J Cancer 2009;45:228-47); Immune-Related Response Criteria (Jedd WE et al., Clin Cancer Res 2009;15(23),7412-7420); and iRANO (Immunotherapy Response Assessment in Neuro-Oncology; Okada H et al., Lancet Oncol 2015 Nov;16(15):e534-42).

### Results:

Chemotherapy as a combination therapy additionally comprising drug-resistant yδ-T cells (and/or drug-resistant natural killer cells), a PARP-1 inhibitor and an inhibitor(s) of immune checkpoint proteins results in an enhanced or prolonged anti-tumor response; a medically beneficial response; additive or synergistic antitumor activity, when compared with chemotherapy alone, or chemotherapy combined with drug-resistant yδ-T cells (and/or drug-resistant natural killer cells); shrinkage in baseline lesions, without new lesions; tumor regression; durable stable disease with possible steady decline in total tumor burden; response after an increase in total tumor burden; and response in the presence of new lesions. Other clinical findings may include an increase in tumor infiltrating lymphocytes as measured by an increase in tumor size; and long-term survival promotion.

For example, temozolomide (TMZ)-treated tumors result in the formation of a significant number of DNA adducts, including N-methylpurines and O⁶-methylguanine. Such adducts result in the activation of the DNA damage response (DDR) and mismatch repair (MMR) machinery. The resistance mechanism to these DNA adducts are through the Base Excision Repair (BER) pathway, which involves PARP-1 to remove N-methylpurine adducts or through the presence of the O⁶ methylguanine DNA methyltransferase (MGMT) protein. O⁶-methylguanine is the major mechanism to TMZ resistance and if MGMT is not present or is inactive, the activation of the MMR process will lead to cross-links, blocked replication forks and the formation of double-stranded DNA breaks (DSB). The DSB results in the formation of MSH-2/MSH-6 dimers and the activation of ataxia telangiectasia Rad3-related (ATR) and ataxia telangiectasia mutated (ATM) kinases.

The activation of this cascade results in the expression of increased numbers of stress ligands on the surface of the tumor cell, such as MICA, MICB, ULBP1-6, and MSH-2. The killing of tumor cells is facilitated by the drug-resistant yδ-T cells and/or drug-resistant natural killer cells whose receptor(s), such as NKG2D, recognizes the tumor-specific stress ligands. However, tumor cells can enzymatically cleave MICA and MICB, releasing soluble MICA and MICB. When bound by effector cell receptors in the plasma, this can lead to down regulation of the receptor expression resulting in immune escape. Chemotherapies such as TMZ have been found to increase cell surface expression of stress ligands on the tumor tissue without significantly increasing the concentration of the soluble ligands in the plasma. PARP-1 inhibitors such as, niraparib, olaparib, or rucaparib have been approved for use in ovarian cancer.

### Methods

### Ex vivo expansion of γδ T cells

Human apheresis products collection from healthy donors. For static culture: All manipulations were performed in an ISO-5 laminar flow hood surrounded by an ISO-7 classified clean room environment. The product was diluted v/v with HBSS (Hank's Balanced Salt Solution) and mononuclear cells isolated by density gradient separation on Ficoll (Sigma-Aldrich; St. Louis, MO). Interphase was harvested and washed X2 in HBSS. The cell pellet was resuspended in CTS^{™} OpTmizer^{™} T Cell Expansion serum free media (ThermoFisher Scientific; Waltham, MA) supplemented with 2mM Zoledronic Acid (Novartis; Basel HV) and 100u/mL IL2 (Miltenyi Biotec Ltd; Bergisch Gladbach, Germany). Cells were cultured in standard T150 or T75 flasks. For closed system culture: All operation including preparation of media, stocks and buffers were performed in ISO 7 clean rooms under biosafety cabinets ion an ISO-t clean room environment using GMP grade reagents. Automated Ficoll separation of the product and cultivation were carried out in CLINIMACS PRODIGY^{®} (Miltenyi Biotec Ltd; Bergisch Gladbach, Germany) bioreactor using customized cell processing program and TS520 tubing set. Cells were then expanded in the CentriCult-Unit of the CLINIMACS PRODIGY^{®} using a combination of Zoledronate (Zometa; Novartis, Basel) and Interleukin-2 (Miltenyi Biotec Ltd; Bergisch Gladbach, Germany) (ZOL/IL-2), in OpTmizer serum-free culture (Thermo Fisher Scientific; Waltham, MA). The culture was kept at a density of 1-2x 10⁶ cells/ml and supplemented with IL-2 100u/mL every other day until cells harvest at day 13. The process was continuously monitored to determine kinetics of expansion and phenotype in comparison with small-scale culture in flasks at regular intervals. Following 9 to 14 days of culture, expanded γδ T cells were phenotyped and cytotoxicity determined against standard leukemia cell lines and glioma tumor cells.

### Example 2-Expression of Stress Ligands on Glioma Cell Lines when Exposed to a Combination of PARP Inhibitor (PARPi) and Temozolomide (TMZ).

### Materials and Methods

### Method of cell collection/processing/culture conditions:

The overall strategy for generating treatment regimen is outlined below. The goal was to generate the total number of γδ T cells required for treating patients with the initial dose administered as a freshly prepared cell population.

Following patient accrual one 2 Volume apheresis collection was obtained following resection and immediately prior to the initiation of induction chemo/radiotherapy.

MNC expansion cultures were initiated at a concentration of 2.0 x 10⁶/ml in the Prodigy^{®} Bioreactor as defined by the validated program and maintained at 5% CO₂ at 37° in OpTimizer (Life Technologies) + 5 µgM Zoledronic Acid (Novartis Oncology; East Hanover, NJ) + 50 u/ml IL-2 (Miltenyi; Duarte, CA). Cultures are maintained for 7 days with addition of 100u/mL IL-2 on post-culture days 2, 5, and 7, and increased to 400u/mL for days +9, +11, and +13. Addition of complete media is determined by pH and cell density.

All cells were culture in the PRODIGY^{®} located in an ISO-7 clean suite with a minimum of 60 air changes/hour. Operations performed outside of the bioreactor will take place in the clean room under an ISO5 biosafety cabinet.

Samples for analysis were obtained pre-expansion and on days 2, 5, 7, 9, 11 and 14 to evaluate cell number and differential. FACS analysis was carried out to determine cell viability and enumerate relevant cellular subsets, including total CD3 T cells, total γδ T cells (including the Vδ2 and Vδ1 subsets), total αβ T cells (including the CD4 and CD8 subsets), as well as NK and B cells. All specimens were analyzed on a FACS Canto^{®} or LSR Fortessa SE^{®} flow cytometer using FACS DiVa^{®} software.

Final product was harvested on day +14, ±3 days. A sample of the final product was counted, analyzed by flow cytometry, and submitted for vector copy number (VCN) and required microbiological testing. The final product will be diluted 1:2 and washed in Plasma-Lyte A and 10% HSA for removal of all remaining manufacturing reagents. After washing and concentration, the product will be resuspended in Plasma-Lyte A, 5% HSA, and 10% DMSO and cryopreserved in 2mL Cryovials at a concentration of 1 x 10⁷/mL until needed.

### Ex vivo modification:

Cell transduction with MGMT Lentivirus (Miltenyi Lentigen: Baltimore, MD) was performed as the culture reaches log expansion stage, normally >20% γδ T Cells. The culture was transduced by spinoculation at MOI of 10 x 2.

Product was thawed from a cryopreserved state and washed in buffered saline and 5% Plasma-Lyte. Post thaw, the final product will be resuspended in the same wash buffer to a concentration of 1x10⁷ γδ T cells.

### Assays:

To measure the putative differential sensitivity of GBM + Temozolomide (TMZ) to PARPi, standardized GBM cell lines LN229, U87MG, and U251MG cells (6000 cells per well of a 96-well plate, plated in triplicate) were exposed to olaparib (5 or 10 mM) alone, olaparib (an exemplary PARP inhibitor)+ 200mg/mL TMZ, or media alone (RPMI + 10% FBS medium) for 4 hours and viability measured at initiation and 8, 16, 24, and 36 hours post treatment using a standardized MTT Viability Assay and incorporation of 7-AAD in a suspension flow cytometry assay.

Data were expressed as %MTT incorporation vs. no treatment following cell solubilization and %7-AAD negative cells for suspension flow cytometry assay. Expression of NKG2D ligands MIC A/B and ULBP 1-6 were also assessed in suspension assay by flow cytometry for each cell line and condition noted above. Data were expressed as log mean fluorescent intensity (MFI) vs. control (media alone) for all time points.

### Results:

The data in **Figures 1-3** show that the combination of a PARP inhibitor and temozolomide (TMZ) act synergistically to upregulate select natural killer group 2D (NKG2D) ligands on three high grade glioma cell lines.

In many cases the NKG2DL maximum increase was transient and expression began to down-regulate after about 24h, also during the optimal treatment window for DRI treatment established with TMZ alone as described in, for example, in Lamb et al., PloS ONE 8(1): e51805. doi:10.1371/journal.pone.0051805.

The patent and scientific literature referred to herein establishes the knowledge that is available to those with skill in the art.

While this invention has been particularly shown and described with references to preferred embodiments thereof, it will be understood by those skilled in the art that various changes in form and details may be made therein without departing from the scope of the invention encompassed by the appended claims. It will also be understood that none of the embodiments described herein are mutually exclusive and may be combined in various ways without departing from the scope of the invention encompassed by the appended claims.

## Claims

1. A composition comprising a population of γδ T cells, for use in a method for treating cancer in a patient in need thereof, the method comprising,
i) administering to said patient a therapeutically effective amount of said composition;
ii) administering to said patient a therapeutically effective amount of a chemotherapeutic agent; and
iii) administering to said patient a therapeutically effective amount of a DDR inhibitor, wherein the DDR inhibitor is a PARP inhibitor.

2. The composition for use of claim 1, wherein the population of γδ T cells is enriched and/or expanded.

3. The composition for use of claim 1, wherein the γδ T cells are genetically engineered to be resistant to at least one chemotherapeutic agent and wherein the chemotherapeutic agent administered to the patient is a chemotherapeutic agent to which the γδ T cells are resistant.

4. The composition for use of claim 3, wherein the PARP inhibitor is administered prior, optionally about 1 day to about 21 days prior, to administering the chemotherapeutic agent and prior to administering the composition.

5. The composition for use of claim 3 or claim 4, wherein the composition is administered about 8 hours to about 72 hours, about 12 hours to about 36 hours, about 8 hours to about 36 hours, or about 24 hours after administration of the chemotherapeutic agent.

6. The composition for use of any one of claims 3 to 5, wherein the composition is administered about 8, 9, 10, 11, 12, 13, 14, 15, 16, 17, 18, 19, 20, 21, 22, 23, 24, 25, 26, 27, 28, 29, 30, 31, 32, 33, 34, 35, or 36 hours after administration of the chemotherapeutic agent.

7. The composition for use of any one of claims 3 to 6, wherein the γδ T-cells have been genetically modified to
(a) encode O⁶ methylguanine DNA methyltransferase (MGMT), alkyl guanine transferase (AGT), P140KMGMT, L22Y-DHFR, thymidylate synthase, dihydrofolate reductase, or multiple drug resistance-1 protein (MDR1),
(b) be resistant to at least two chemotherapeutic agents selected from: is an alkylating agent; a metabolic antagonist; a DNA demethylating agent; a substituted nucleotide; a substituted nucleoside; an antitumor antibiotic; a plant-derived antitumor agent and a nitrosurea, or
(c) be resistant to at least two chemotherapeutic agents selected from temozolomide; cisplatin; carboplatin; etoposide; methotrexate (MTX); trimethotrexate (TMTX); dacarbazine (DTIC), raltitrexed; S-(4-Nitrobenzyl)-6-thioinosine (NBMPR); 6-benzyguanidine (6-BG); a nitrosourea (rabinopyranosyl-N-methyl-N-nitrosourea (Aranose), Carmustine (BCNU, BiCNU), Chlorozotocin, Ethylnitrosourea (ENU), Fotemustine, Lomustine (CCNU), Nimustine, N-Nitroso-N-methylurea (NMU), Ranimustine (MCNU), Semustine, Streptozocin (Streptozotocin)); cytarabine; camptothecin; and a therapeutic derivative of any thereof.

8. The composition for use according to any preceding claim, wherein the PARP inhibitor is selected from the group consisting of niraparib, olaparib, rucaparib, talazoparib, and veliparib.

9. The composition for use according to any preceding claim, wherein the chemotherapeutic agent is
(a) an alkylating agent; a metabolic antagonist; a DNA demethylating agent; a substituted nucleotide; a substituted nucleoside; an antitumor antibiotic; a plant-derived antitumor agent or a nitrosourea,
(b) selected from temozolomide; cisplatin; carboplatin; etoposide; methotrexate (MTX); trimethotrexate (TMTX);dacarbazine (DTIC), raltitrexed; S-(4-Nitrobenzyl)-6-thioinosine (NBMPR); 6-benzyguanidine (6-BG); a nitrosourea (rabinopyranosyl-N-methyl-N-nitrosourea (Aranose), Carmustine (BCNU, BiCNU), Chlorozotocin, Ethylnitrosourea (ENU), Fotemustine, Lomustine (CCNU), Nimustine, N-Nitroso-N-methylurea (NMU), Ranimustine (MCNU), Semustine, Streptozocin (Streptozotocin)); cytarabine; camptothecin; and a therapeutic derivative of any thereof, or
(c) TMZ, methotrexate, DTIC, BCNU, CCNU, MCNU, NMU or ENU.

10. The composition for use of any preceding claim, wherein the method further comprises the step of administering an immune checkpoint inhibitor (ICP).

11. The composition for use of claim 10, wherein the immune checkpoint inhibitor targets PD1, CTLA-4, PDL1, PDL2, B7-H3, B7-H4, BTLA, HVEM, TIM3, GAL9, LAG3, VISTA, KIR, 2B4, CD160 (also referred to as BY55), CGEN-15049, CHK 1 kinase, CHK2 kinase, A2aR, OX40, or a B-7 family ligand, preferably PD-1, CTLA-4, PDL1 or PDL2.

12. The composition for use of any preceding claim, wherein the cancer is selected from: ovarian cancer, central nervous system (CNS) tumors, melanoma, uveal melanoma, neuroendocrine tumors, adrenal tumors, non-Hodgkin's lymphoma, soft tissue sarcomas, bone cancer, uterine sarcoma, small lung cancer (SCLC) and Zollinger-Ellison syndrome.

13. The composition for use of any preceding claim, wherein the composition comprises greater than about 60% γδ T cells, less than about 5% αβ T cells and less than about 25% natural killer (NK) cells.

14. The composition for use of any preceding claim, wherein the composition comprises about 5 x 10⁸ γδ T cells/kg or less of the patient's weight, about 5 x 10⁷ γδ T cells/kg or less of the patient's weight, or about 5 x 10⁶ γδ T cells/kg or less of the patient's weight.

15. The composition for use of any preceding claim, wherein the chemotherapeutic agent is administered prior to administering the composition.

## Patentansprüche

1. Zusammensetzung, umfassend eine Population von γδ-T-Zellen, zur Verwendung bei einem Verfahren zur Behandlung von Krebs bei einem behandlungsbedürftigen Patienten, wobei das Verfahren
i) Verabreichen einer therapeutisch wirksamen Menge der Zusammensetzung an den Patienten umfasst;
ii) Verabreichen einer therapeutisch wirksamen Menge eines Chemotherapeutikums an den Patienten umfasst; und
iii) Verabreichen einer therapeutisch wirksamen Menge eines DDR-Inhibitors an den Patienten umfasst, wobei es sich bei dem DDR-Inhibitor um einen PARP-Inhibitor handelt.

2. Zusammensetzung zur Verwendung nach Anspruch 1, wobei die Population von γδ-T-Zellen angereichert und/oder expandiert ist.

3. Zusammensetzung zur Verwendung nach Anspruch 1, wobei die γδ-T-Zellen gentechnisch so konstruiert sind, dass sie gegen mindestens ein Chemotherapeutikum resistent sind, und wobei es sich bei dem dem Patienten verabreichten Chemotherapeutikum um ein Chemotherapeutikum handelt, gegen das die γδ-T-Zellen resistent sind.

4. Zusammensetzung zur Verwendung nach Anspruch 3, wobei der PARP-Inhibitor vor, gegebenenfalls etwa 1 Tag bis etwa 21 Tage vor, Verabreichen des Chemotherapeutikums und vor Verabreichen der Zusammensetzung verabreicht wird.

5. Zusammensetzung zur Verwendung nach Anspruch 3 oder Anspruch 4, wobei die Zusammensetzung etwa 8 Stunden bis etwa 72 Stunden, etwa 12 Stunden bis etwa 36 Stunden, etwa 8 Stunden bis etwa 36 Stunden oder etwa 24 Stunden nach Verabreichung des Chemotherapeutikums verabreicht wird.

6. Zusammensetzung zur Verwendung nach einem der Ansprüche 3 bis 5, wobei die Zusammensetzung etwa 8, 9, 10, 11, 12, 13, 14, 15, 16, 17, 18, 19, 20, 21, 22, 23, 24, 25, 26, 27, 28, 29, 30, 31, 32, 33, 34, 35 oder 36 Stunden nach Verabreichung des Chemotherapeutikums verabreicht wird.

7. Zusammensetzung zur Verwendung nach einem der Ansprüche 3 bis 6, wobei die γδ-T-Zellen gentechnisch verändert wurden, so dass sie
(a) O⁶-Methylguanin-DNA-Methyltransferase (MGMT), Alkylguanin-Transferase (AGT), P140KMGMT, L22Y-DHFR, Thymidylat-Synthase, Dihydrofolat-Reduktase oder MDR1 (Multiple Drug Resistance-1 Protein) codieren,
(b) gegen mindestens zwei Chemotherapeutika resistent sind, die aus einem Alkylierungsmittel, einem metabolischen Antagonisten, einem DNA-Demethylierungsmittel, einem substituierten Nukleotid, einem substituierten Nukleosid, einem Antitumorantibiotikum, einem pflanzlichen Antitumormittel und einem Nitrosoharnstoff ausgewählt sind, oder
(c) gegen mindestens zwei Chemotherapeutika resistent sind, die aus Temozolomid, Cisplatin, Carboplatin, Etoposid, Methotrexat (MTX), Trimethotrexat (TMTX), Dacarbazin (DTIC), Raltitrexed, S-(4-Nitrobenzyl)-6-thioinosin (NBMPR), 6-Benzyguanidin (6-BG), einem Nitrosoharnstoff (Rabinopyranosyl-N-methyl-N-nitrosoharnstoff (Aranose), Carmustin (BCNU, BiCNU), Chlorozotocin, Ethylnitrosoharnstoff (ENU), Fotemustin, Lomustin (CCNU), Nimustin, N-Nitroso-N-methylharnstoff (NMU), Ranimustin (MCNU), Semustin, Streptozocin (Streptozotocin)), Cytarabin, Camptothecin und irgendeinem therapeutischen Derivat davon ausgewählt sind.

8. Zusammensetzung zur Verwendung gemäß einem vorhergehenden Anspruch, wobei der PARP-Inhibitor aus der Gruppe bestehend aus Niraparib, Olaparib, Rucaparib, Talazoparib und Veliparib ausgewählt ist.

9. Zusammensetzung zur Verwendung gemäß einem vorhergehenden Anspruch, wobei das Chemotherapeutikum
(a) ein Alkylierungsmittel, ein metabolischer Antagonist, ein DNA-Demethylierungsmittel, ein substituiertes Nukleotid, ein substituiertes Nukleosid, ein Antitumorantibiotikum, ein pflanzliches Antitumormittel oder ein Nitrosoharnstoff ist,
(b) aus Temozolomid, Cisplatin, Carboplatin, Etoposid, Methotrexat (MTX), Trimethotrexat (TMTX), Dacarbazin (DTIC), Raltitrexed, S-(4-Nitrobenzyl)-6-thioinosin (NBMPR), 6-Benzyguanidin (6-BG), einem Nitrosoharnstoff (Rabinopyranosyl-N-methyl-N-nitrosoharnstoff (Aranose), Carmustin (BCNU, BiCNU), Chlorozotocin, Ethylnitrosoharnstoff (ENU), Fotemustin, Lomustin (CCNU), Nimustin, N-Nitroso-N-methylharnstoff (NMU), Ranimustin (MCNU), Semustin, Streptozocin (Streptozotocin)), Cytarabin, Camptothecin und irgendeinem therapeutischen Derivat davon ausgewählt ist oder
(c) TMZ, Methotrexat, DTIC, BCNU, CCNU, MCNU, NMU oder ENU ist.

10. Zusammensetzung zur Verwendung nach einem vorhergehenden Anspruch, wobei das Verfahren ferner den Schritt des Verabreichens eines Immuncheckpoint-Inhibitors (ICP) umfasst.

11. Zusammensetzung zur Verwendung nach Anspruch 10, wobei der Immuncheckpoint-Inhibitor auf PD1, CTLA-4, PDL1, PDL2, B7-H3, B7-H4, BTLA, HVEM, TIM3, GAL9, LAG3, VISTA, KIR, 2B4, CD160 (auch als BY55 bezeichnet), CGEN-15049, CHK1-Kinase, CHK2-Kinase, A2aR, OX40 oder einen Liganden der B-7-Familie, vorzugsweise PD-1, CTLA-4, PDL1 oder PDL2, zielt.

12. Zusammensetzung zur Verwendung nach einem vorhergehenden Anspruch, wobei der Krebs ausgewählt ist aus:
Ovarialkarzinom, Tumoren des zentralen Nervensystems (ZNS), Melanom, Aderhautmelanom, neuroendokrinen Tumoren, Nebennierentumoren, Non-Hodgkin-Lymphom, Weichteilsarkomen, Knochenkrebs, Uterussarkom, kleinzelligem Lungenkrebs (SCLC) und Zollinger-Ellison-Syndrom.

13. Zusammensetzung zur Verwendung nach einem vorhergehenden Anspruch, wobei die Zusammensetzung mehr als etwa 60 % γδ-T-Zellen, weniger als etwa 5 % αβ-T-Zellen und weniger als etwa 25 % NK(Natural Killer)-Zellen umfasst.

14. Zusammensetzung zur Verwendung nach einem vorhergehenden Anspruch, wobei die Zusammensetzung etwa 5 x 10⁸ γδ-T-Zellen/kg oder weniger des Gewichts des Patienten, etwa 5 x 10⁷ γδ-T-Zellen/kg oder weniger des Gewichts des Patienten oder etwa 5 x 10⁶ γδ-T-Zellen/kg oder weniger des Gewichts des Patienten umfasst.

15. Zusammensetzung zur Verwendung nach einem vorhergehenden Anspruch, wobei das Chemotherapeutikum vor Verabreichen der Zusammensetzung verabreicht wird.

## Revendications

1. Composition comprenant une population de cellules T γδ, destinée à être utilisée dans un procédé de traitement du cancer chez un patient qui en a besoin, le procédé comprenant
i) administrer audit patient une quantité thérapeutiquement efficace de ladite composition;
ii) administrer audit patient une quantité thérapeutiquement efficace d'un agent chimiothérapeutique ; et
iii) administrer audit patient une quantité thérapeutiquement efficace d'un inhibiteur de DDR, l'inhibiteur de DDR étant un inhibiteur de PARP.

2. Composition pour utilisation selon la revendication 1, dans laquelle la population de cellules T γδ est enrichie et/ou expansée.

3. Composition pour utilisation selon la revendication 1, dans laquelle les cellules T γδ sont génétiquement modifiées pour être résistantes à au moins un agent chimiothérapeutique et dans laquelle l'agent chimiothérapeutique administré au patient est un agent chimiothérapeutique auquel les cellules T γδ sont résistantes.

4. Composition pour utilisation selon la revendication 3, dans laquelle l'inhibiteur de PARP est administré avant, facultativement environ 1 jour à environ 21 jours avant, l'administration de l'agent chimiothérapeutique et avant l'administration de la composition.

5. Composition pour utilisation selon la revendication 3 ou la revendication 4, dans laquelle la composition est administrée environ 8 heures à environ 72 heures, environ 12 heures à environ 36 heures, environ 8 heures à environ 36 heures, ou environ 24 heures après administration de l'agent chimiothérapeutique.

6. Composition pour utilisation selon l'une quelconque des revendications 3 à 5, dans laquelle la composition est administrée environ 8, 9, 10, 11, 12, 13, 14, 15, 16, 17, 18, 19, 20, 21, 22, 23, 24, 25, 26, 27, 28, 29, 30, 31, 32, 33, 34, 35 ou 36 heures après l'administration de l'agent chimiothérapeutique.

7. Composition pour utilisation selon l'une quelconque des revendications 3 à 6, dans laquelle les cellules T γδ ont été génétiquement modifiés pour
(a) coder pour O⁶ méthylguanine ADN méthyltransférase (MGMT), alkylguanine transférase (AGT), P140KMGMT, L22Y-DHFR, thymidylate synthase, dihydrofolate réductase, ou protéine de résistance multiple aux médicaments 1 (MDR1),
(b) être résistantes à au moins deux agents chimiothérapeutiques choisis parmi : un agent alkylant ; un antagoniste métabolique ; un agent de déméthylation d'ADN ; un nucléotide substitué ; un nucléoside substitué ; un antibiotique antitumoral ; un agent antitumoral dérivé de plante et une nitrosurée, ou
c) être résistantes à au moins deux agents chimiothérapeutiques choisis parmi témozolomide ; cisplatine ; carboplatine ; étoposide ; méthotrexate (MTX) ; triméthotrexate (TMTX) ; dacarbazine (DTIC) ; raltitrexed ; S-(4-nitrobenzyl)-6-thioinosine (NBMPR) ; 6-benzyguanidine (6-BG) ; une nitrosourée (rabinopyranosyl-N-méthyl-N-nitrosourée (Aranose), carmustine (BCNU, BiCNU), chlorozotocine, éthylnitrosourée (ENU), fotémustine, lomustine (CCNU), nimustine, N-nitroso-N-méthylurée (NMU), ranimustine (MCNU), semustine, streptozocine (Streptozotocine)) ; cytarabine ; camptothécine ; et un dérivé thérapeutique de l'un quelconque de ceux-ci.

8. Composition pour utilisation selon l'une quelconque des revendications précédentes, dans laquelle l'inhibiteur de PARP est choisi dans le groupe constitué par niraparib, olaparib, rucaparib, talazoparib, et véliparib.

9. Composition pour utilisation selon l'une quelconque des revendications précédentes, dans laquelle l'agent chimiothérapeutique est
(a) un agent alkylant ; un antagoniste métabolique ; un agent déméthylant l'ADN ; un nucléotide substitué ; un nucléoside substitué ; un antibiotique antitumoral ; un agent antitumoral d'origine végétale ou une nitrosourée,
(b) choisi parmi témozolomide ; cisplatine ; carboplatine ; étoposide ; méthotrexate (MTX) ; triméthotrexate (TMTX) ; dacarbazine (DTIC) ; raltitrexed ; S-(4-nitrobenzyl)-6-thioinosine (NBMPR) ; 6-benzylguanidine (6-BG) ; une nitrosourée (rabinopyranosyl-N-méthyl-N-nitrosourée (Aranose), carmustine (BCNU, BiCNU), chlorozotocine, éthylnitrosourée (ENU), fotémustine, lomustine (CCNU), nimustine, N-nitroso-N-méthylurée (NMU), ranimustine (MCNU), semustine, streptozocine (Streptozotocine)) ; cytarabine ; camptothécine ; et un dérivé thérapeutique de l'un de ces médicaments, ou
(c) TMZ, méthotrexate, DTIC, BCNU, CCNU, MCNU, NMU ou ENU.

10. Composition pour utilisation selon l'une quelconque des revendications précédentes, dans laquelle le procédé comprend en outre l'étape d'administration d'un inhibiteur de point de contrôle immunitaire (ICP).

11. Composition pour utilisation selon la revendication 10, dans laquelle l'inhibiteur de point de contrôle immunitaire cible PD1, CTLA-4, PDL1, PDL2, B7-H3, B7-H4, BTLA, HVEM, TIM3, GAL9, LAG3, VISTA, KIR, 2B4, CD160 (également appelé BY55), CGEN-15049, CHK 1 kinase, CHK2 kinase, A2aR, OX40 ou un ligand de la famille B-7, de préférence PD-1, CTLA-4, PDL1 ou PDL2.

12. Composition pour utilisation selon l'une quelconque des revendications précédentes, dans laquelle le cancer est choisi parmi :
cancer de l'ovaire, tumeurs du système nerveux central (SNC), mélanome, mélanome uvéal, tumeurs neuroendocrines, tumeurs surrénaliennes, lymphome non hodgkinien, sarcomes des tissus mous, cancer des os, sarcome utérin, cancer du poumon à petites cellules (CPPC) et syndrome de Zollinger-Ellison.

13. Composition pour utilisation selon l'une quelconque des revendications précédentes, dans laquelle la composition comprend plus d'environ 60 % de cellules T γδ, moins d'environ 5 % de cellules T αβ et moins d'environ 25 % de cellules tueuses naturelles (NK).

14. Composition pour utilisation selon l'une quelconque des revendications précédentes, dans laquelle la composition comprend environ 5 x 10⁸ γδ cellules T/kg ou moins du poids du patient, environ 5 x 10⁷ γδ cellules T/kg ou moins du poids du patient, ou environ 5 x 10⁶ γδ cellules T/kg ou moins du poids du patient.

15. Composition pour utilisation selon l'une quelconque des revendications précédentes, dans laquelle l'agent chimiothérapeutique est administré avant l'administration de la composition.
